Europäisches Patentamt

(19) European Patent Office (11) Publication number: **0 022 227**

Office européen des brevets **B1**

(12) **EUROPEAN PATENT SPECIFICATION**

(45) Date of publication of patent specification: **26.09.84** (51) Int. Cl.³: **B 32 B 27/12,** A 41 B 13/02,
A 61 F 13/18

(21) Application number: **80103652.6**

(22) Date of filing: **27.06.80**

(54) **Flexible absorbent laminates and process for making them.**

<table>
<tr><td>(30) Priority: <b>09.07.79 US 55586</b></td><td>(73) Proprietor: <b>THE DOW CHEMICAL COMPANY<br>Dow Center 2030 Abbott Road Post Office Box<br>1967<br>Midland Michigan 48640 (US)</b></td></tr>
<tr><td>(43) Date of publication of application:<br><b>14.01.81 Bulletin 81/02</b></td><td></td></tr>
<tr><td>(45) Publication of the grant of the patent:<br><b>26.09.84 Bulletin 84/39</b></td><td>(72) Inventor: <b>Erickson, Robert Everett<br>5413 Nurmi Drive<br>Midland Michigan (US)</b></td></tr>
<tr><td>(84) Designated Contracting States:<br><b>AT BE DE FR GB IT SE</b></td><td>(74) Representative: <b>Casalonga, Axel<br>BUREAU D.A. CASALONGA OFFICE JOSSE &amp;<br>PETIT Baaderstrasse 12-14<br>D-8000 München 5 (DE)</b></td></tr>
<tr><td>(56) References cited:<br><b>FR-A-2 173 047<br>US-A-3 670 731<br>US-A-3 890 974<br>US-A-4 076 673<br>US-A-4 117 184</b><br><br><b>The file contains technical information<br>submitted after the application was filed and<br>not included in this specification</b></td><td></td></tr>
</table>

Courier Press, Leamington Spa, England.

## 0 022 227

**Description**

This invention relates to flexible absorbent laminates wherein a cross-linked polyelectrolyte film is bonded to wicking substrates, dried, and crushed to give laminate flexibility and high water absorption rates.

It is known from U.S. Patent 3,669,822, Cowen, June 13, 1972, that tissue/polyethylene film/tissue laminates can be crimped or embossed to give an improved hand or flexibility or cloth-like feel.

It is also known from French Patent 2,375,985, September 1, 1978, that non-woven fiber sheet/tissue/absorbent layer/tissue/polyethylene film laminates can be made flexible with adhesion to the film by adding an adhesive between the tissue end and the polyethylene film followed by transverse creasing or crimping.

In U.S. Patent 4,117,184, Erickson, September 26, 1978, it is disclosed that tissue/aerated absorbent film/tissue laminates can be prepared with improved absorption rates.

While the laminates disclosed in US patent 4 117 184 have good absorption rates for water, urine, and other body fluids or exudates they have a tendency to become brittle and inflexible in atmospheres of low relative humidity. The result is an unacceptable rattling sound when the laminate is flexed and the laminate has a stiff or board-like feel.

It has now been found, according to the present invention, that laminates can be prepared which are both highly absorbent and flexible at both high and low relative humidities.

The present invention is a flexible hydrophilic absorbent laminate comprising a central film consisting of a crosslinked water-swellable polyelectrolyte that is water-soluble in the salt form and a layer of wicking substrates bonded to both sides of said film, characterized in that
— said film is discontinuous, crushed and laminated to said wicking substrates, and
— said flexible absorbent laminate is flexible at low and high relative humidity and is obtainable by a process consisting in passing the laminate, if necessary dried to less than 8 wt.% residual moisture, through a crushing zone wherein the said film breaks into a plurality of pieces which remain laminated to said substrates.

The present invention also is directed to the laminate described above and further characterized in that the polyelectrolyte is a lightly cross-linked carboxylated polyelectrolyte.

While the absorbent film can be a solid film as in US patent 4 076 673 Burkholder, Feb. 28, 1978, it is preferably an aerated film as disclosed in US patent 4 117 184.

The present invention also embraces a process for making the flexible laminate described above, comprising

(a) reducing the moisture content of a laminate of a film, prepared from a cross-linked water-swellable polyelectrolyte that is water-soluble in the salt form, with wicking substrates to less than 8 percent by weight by passing said laminate through a drying zone; and

(b) passing said dried laminate through a crushing zone wherein said film breaks into a plurality of pieces which remain laminated to said substrates.

The laminates are useful to make absorbent articles such as baby diapers, or adult diapers for incontinent patients, since the laminates and/or articles readily absorb aqueous solutions such as blood, urine, and other body exudates. The absorbent articles contain one or more layers of the laminate and non-woven fiber mats or cellulose fluff, together with a water impermeable bottom sheet such as polyethylene and a water permeable top sheet such as non-woven fiber mat.

The drawing is a photographic reproduction of one species of the present invention.

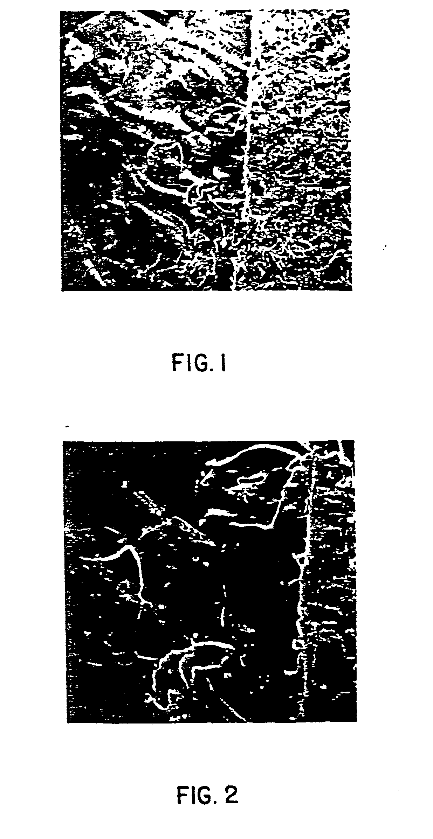

Figure 1 shows a view taken with a scanning electron microscope of the laminate with the top layer of paper tissue fibers partially pulled back to expose the crushed film underneath. The bottom layer of paper tissue fibers is clearly evident beneath the crushed film.

Figure 2 is an enlarged view of the center portion of Figure 1, showing in greater detail the craters and bubbles in the aerated film and the bonding of the film to the fibers.

The polyelectrolytes used in this invention must be water-soluble in the salt form. Examples of useful polyelectrolytes include ammonium or alkali metal salts of homopolymers of acrylic or methacrylic acid and copolymers with one or more ethylenically unsaturated comonomers.

Preferably the polyelectrolyte is a partially saponified polyacrylate carboxylic polymer. The polymer before saponification is the result of reacting a mixture of monomers which comprises (1) 30 to 92 percent by weight of an alkyl acrylate wherein the alkyl group has from 1 to 10 carbon atoms, an alkyl methacrylate wherein the alkyl group has from 4 to 10 carbon atoms, or mixtures thereof; (2) 8 to 70 percent by weight of an olefinically unsaturated carboxylic acid; and (3) 0 to 15 percent by weight of an omega-hydroxyalkyl acrylate wherein the hydroxyalkyl groups have from 1 to 4 carbon atoms. Also included as a reactant is a polyfunctional or difunctional cross-linking agent. By use of the term "polyfunctional cross-linking agent" is meant a cross-linking agent having three or more reactive sites available for cross-linking.

Example of useful alkyl acrylates include methyl acrylate, ethyl acrylate, propyl acrylate, butyl acrylate and hexyl acrylate. Examples of useful alkyl methacrylates include methyl methacrylate, ethyl methacrylate, hexyl methacrylate, n-octyl methacrylate and n-decyl methacrylate. Examples of useful omega-hydroxyalkyl acrylates include 2-hydroxyethyl acrylate, hydroxymethyl acrylate, 3-hydroxy-

2

propyl acrylate and 4-hydroxybutyl acrylate.

The olefinically unsaturated carboxylic acids useful in this invention are mono- or polycarboxylic acids. Examples of monocarboxylic acids include acrylic acid, methacrylic acid, crotonic acid, and isocrotonic acid. Examples of polycarboxylic acids include maleic acid, fumaric acid, and itaconic acid.

The foregoing polyacrylates are then dissolved in an aqueous alkali metal hydroxide solution. The amount of hydroxide solution employed is sufficient to saponify some of the polymerized acrylate esters to alkali metal carboxylates and to neutralize the carboxylic groups of the polyacrylate to alkali metal carboxylates so that the saponified polyacrylate polymer has from 30 to 70 weight percent alkali metal carboxylates.

The partially saponified polyacrylate polymer is employed as a solution containing from 5 to 60 percent by weight of the polymer.

Other types of acrylic copolymers that can be employed to make the polyelectrolytes for the present invention include copolymers of acrylic esters or acrylic acids with starch or other polysaccharides, monosaccharides such as glucose, mannose or galactose, and disaccharides such as sucrose.

Illustrative examples of the polyfunctional cross-linking agents useful in this invention are set forth in U.S. Patents 2,926,154; 3,224,986; and 3,332,901. These polyfunctional cross-linking agents are generally known as polyamide-polamine epichlorohydrin adducts. The structure of these adducts has been discussed in an article by M. E. Carr et al., *Journal of Applied Polymer Science*, Vol. 17, pages 721—735 (1973).

Illustrative examples of the difunctional cross-linking agents useful in this invention are poly-haloalkanols such as 1,3-dichloroisopropanol; 1,3-dibromoisopropanol; sulfonium zwitterions such as the tetrahydrothiophene adduct of novolac resins; haloepoxyalkanes such as epichlorohydrin, epibromohydrin, 2-methyl epichlorohydrin and epiiodohydrin; polyglycidyl ethers such as 1,4-butanediol diglycidyl ether, glycerine-1,3-diglycidyl ether, ethylene glycol diglycidyl ether, propylene glycol diglycidyl ether, diethylene glycol diglycidyl ether, neopentylglycol diglycidyl ether, polypropylene glycol diglycidyl ethers having an epoxy equivalent weight range from about 175 to about 380, bisphenol A-epichlorohydrin epoxy resins having an epoxy equivalent weight range from about 182 to about 975, and mixtures of the foregoing.

Also useful as cross-linking agents are monomeric amine-epihalohydrin adducts prepared by reacting at least two moles of an epihalohydrin with one mole of various monoamines, diamines or triamines, at a temperature in the range from 0°C to 90°C for 0.5 to 8 hours. The reaction is carried out in a reaction medium containing 10—20 weight percent polymer solids, and the remainder water, a lower alcohol such as methanol or ethanol, or aqueous solutions of the lower aliphatic alcohols, i.e., those containing 1—4 carbon atoms, can be used. The amine-epihalohydrin adducts are used directly as made without separation or concentration.

Sulfonium zwitterions are known from U.S. Patents 3,660,431; 3,749,737 and 3,749,738.

These cross-linking agents are used in an amount from about 0.05 to about 5.0 percent based on the weight of the polyelectrolyte used. This is generally sufficient to cause the polyelectrolyte to become lightly cross-linked.

It is sometimes desirable to add a small amount of a surfactant to the polyelectrolyte composition to aid in flowing on and removing the continuous film from the water impervious substrate. A secondary benefit of using a surfactant is to increase the wettability of the final dry absorbent film. Either anionic or nonionic surfactants may be used. Examples of the useful surfactants are the sodium alkyl sulfonates and the ethylene oxide derivatives of alkylated phenols.

For the purpose of this invention, a moisture- or water-absorbent or water-swellable polyelectrolyte is defined as one which absorbs greater than about 15 times its weight of synthetic or natural urine. Preferably the absorbency should be in the range from about 30—60 grams of urine per gram of polyelectrolyte or in the range of 90—250 grams of deionized water per gram of polyelectrolyte. The amount of cross-linking agent used is a variable factor which is dependent upon the particular polyelectrolyte used and the molecular weight of the polyelectrolyte. Preferably, the amount used varies from 0.25 to 3.0 percent based on the weight of the polyelectrolyte. However, this range is varied for each polyelectrolyte in order to adjust the absorbency of the final cross-linked gel.

The water-swellable laminates of this invention may be combined into absorbent pads with wicking or non-wicking substrates. Examples of wicking substrates include woven fabrics, non-woven fiber mats, cellulose fluff, polymer foams, tissue paper, crepe paper and paper toweling.

The flexible laminates of this invention are made by first reducing the moisture content of the aerated laminates prepared by U.S. Patent 4,117,184 in a drying zone. It is to be understood that similar flexible laminates can be made using the non-aerated laminates or films of U.S. Patent 4,076,673.

The moisture content of the laminates must be reduced from their normal content of about 14 percent moisture to less than 8 percent and preferably in the range from 1—6 percent. If the moisutre content is greater than about 8 percent, there is substantially no crushing or shattering of the film in the subsequent cracking zone since the film remains flexible. If the moisture content is less than 1 percent moisture, there is substantial tearing or rupturing of the wicking substrates, such as the fibers of the

3

# O 022 227

paper tissue mats.

The above drying step can be accomplished with conventional drying equipment such as a steam heated drying drum, microwave heaters, infrared heaters, or similar equipment.

The second step of the process involves passing the dried laminate through a crushing zone wherein the film breaks into a plurality of pieces which remain substantially bonded to the substrates. This can be accomplished by passing the dried laminate together with a wire mesch screen through a pair of rollers where one of the rollers should have a yieldable or flexible surface. Suitable rollers are commercially available. They have an elastomeric surface with a Durometer A hardness from about 40 to about 100 and preferably in the range from 50 to 70. Generally these rollers are made from synthetic rubber such as neoprene, but other elastomeric materials such as polyvinyl chloride and polyurethane surfaces are acceptable provided that they have the required Durometer hardness.

A preferred method is to use a yieldable roller coacting with a second steel roller having an engraved or etched pattern on the surface thereof. The choice of the pattern is not critical as long as it gives a patterned imprint to the laminate. It is to be noted that merely embossing the laminate does not improve its hand and/or absorption. There must be a substantial amount of film breakage.

There are several variables in the crushing zone which must be considered in order to obtain an acceptable product. The degree of moisture in the laminate, the degree of pressure between the rolls, and the relative hardness of the yieldable roll are all interrelated. For example, if the yieldable roll is too soft and the pressure is too great, there will be substantial amounts of tearing of the substrates. At the other extreme, with a relatively hard, yieldable roll and little pressure there will be little or no film crushing and thus little or no flexibility in the final laminates.

By suitable adjustments in the foregoing variables, one can obtain a sufficient crushing of the film within the laminate without tearing or destroying the wicking substrates which are bonded to the film.

The following examples illustrate the invention.

## Examples 1—3

Various samples of a paper/aerated film/paper laminate having the dimensions 3 × 12 inches (7.62 × 30.5 cm) and prepared by the method set forth in Example 9 of U.S. Patent 4,117,184 were dried on a steam heated drum roll at 230°F—240°F (110°C—115.6°C) for varying lengths of time. The samples each had a moisture content of 8—13 percent by weight prior to drying. Each sample was then combined with a 16 mesh stainless steel wire mesh screen and run through a pair of calender rolls at a rate of twenty feet (6.1 m) per minute with a pressure of 40—50 psig (2.82—3.52 kg/cm²). The upper roll was a 3-inch diameter by an 18-inch (7.62 × 45.7 cm) long steel roll with a synthetic rubber surface 1/2 inch (1.27 cm) deep and having a 78 to 80 Shore A Durometer hardness. The lower roll was a relatively smooth steel roll of 5 inches diameter by 18 inches long (12.7 × 45.7 cm).

Two control runs were also conducted under the same conditions except that the samples were not subjected to heat and crushing.

The samples were then compared and observed for their softness or hand at 30—40 percent relative humidity on an arbitrary scale of 1 to 10 where 1 represents the high stiffness of writing paper and the loud rattle of cellophane and 10 represents the low stiffness and the soft rustle of facial tissue paper. Any rating of 7 or more is considered satisfactory.

The results are set forth in Table I below:

### TABLE I

| Experiment | Contact with hot drum (seconds) | Percent Moisture in Dried Laminate | Softness Rating |
|---|---|---|---|
| Control 1 | 0 | 10—13 | 3 |
| Control 2 | 0 | 8—9 | 5 |
| Example 1 | 3 | 6 | 7 |
| Example 2 | 5 | 4 | 9 |
| Example 3 | 15 | <1 | 9 |

Examples 4—7

Following the procedure set forth in Example 2, where the samples were held in contact with the hot drum for 5 seconds, the wire mesh was replaced with screens having a variety of mesh sizes. The results are set forth in Table II.

TABLE II

| Example | Mesh | Screen Wire Dia., Inches (mm) | Percent Moisture in Dried Laminate | Rating |
|---|---|---|---|---|
| 4 | 24 | .014 (0.36) | 4 | 8 |
| 5 | 16 | .023 (0.58) | 4 | 9 |
| 6 | 12 | .041 (1.04) | 4 | 8 |
| 7 | 8 | .025 (0.63) | 4 | 8 |

These examples show that the diameter of the wire has no effect on the moisture content or on the final rating of the laminate.

Examples 8—10

A roll of paper/aerated film/paper laminate was prepared according to U.S. Patent 4,117,184 and had the following characteristics: total weight 7.1 grams per square foot (76.5 $g/m^2$), paper tissue weight 3.3 grams per square foot (35.6 $g/m^2$), film weight 3.8 grams per square foot (41.0 $g/m^2$) and film moisture content 14 percent.

The laminate was unwound from the feed roll and dried on a steam heated rotating drum having a surface temperature of 220°F—230°F (104.4°C—110°C) and with variable speed and take off times so as to obtain variable drying times.

The laminate was immediately fed at 20 feet (6.10 m) per minute to a pair of calendar rolls having a pressure of 40—50 psig (2.82—3.52 $kg/cm^2$) as in Example 1 except that the lower roll was tightly covered with a cylindrical cloth screen of 16 mesh with .023 inch (0.58 mm) diameter wires. The results are set forth in Table III.

TABLE III

| Example | Drum, RPM | Laminate Contact Time, sec. | Dried Laminate, Percent Moisture | Softness Rating |
|---|---|---|---|---|
| 8 | 9 | 19 | 3.6 | 9 |
| 9 | 15 | 15 | 4.3 | 9 |
| 10 | 15 | 12 | 5.5 | 8 |
| Control 3 | 15 | 10 | 8.0 | 5 |
| Control 4 | (no drying) | | 14.0 | 3 |

Control 3 shows that a final moisture content of 8 percent will not produce a satisfactory softness rating. The above examples were tested for their absorption rate by the following test:

A sample of the laminate on a glass plate was placed under a 6 × 6 inch (15.2 cm × 15.2 cm) square metal box having 0.25 inch (0.64 cm) thick walls. The box was weighted with lead weights on each corner to prevent leakage. Since the capacity of film to absorb water in the laminate (grams of saline solution per gram of film) and the weight of the film per square foot (0.093 $m^2$) is known, it can be calculated what the capacity of the laminate sample will be. The saline solution used is 1 percent sodium chloride in water to simulate urine.

Into this box is poured 75 percent of the capacity of sample and a timer is started. When the liquid is absorbed completely as shown by lack of gloss due to free liquid, the timer is stopped. The time in

5

seconds is the absorption rate.

The test results are given in Table IV.

TABLE IV

| Examples | Laminate Treatment | Absorption Rate, (Seconds) |
|---|---|---|
| Control 4 | none | 1300 |
| Example 8 | crushed | 75—80 |
| Example 9 | crushed | 80—90 |
| Example 10 | crushed | 80—90 |

## Example 11

A laminate similar to that of Example 8 was prepared having the following characteristics: total weight 6.3 grams per square foot (68.0 g/m²); tissue weight 2.6 grams per square foot (28.0 g/m²); film weight 3.7 grams per square foot (39.9 g/m²) and 13—15 percent moisture.

This laminate was dried to 4 percent moisture and crushed as set forth in Example 8 to a nip pressure of 40—50 psig (2.82—3.52 kg/cm²). The flexible crushed laminate had a softness rating of 7.

## Example 12

The procedure of Example 11 was repeated using a pressure of 75 psig (5.30 kg/cm²). The resulting flexible cracked laminate had an increased softness rating of 9.

## Example 13

The procedure of Example 9 was repeated using microwave heating and radiant (infrared) heating in place of the steam heated drum. The same softness rating was obtained.

## Example 14

An absorbent laminate containing a film weight of 3 grams per square foot (32.4 g/m²) and a tissue weight of 2.8 grams per square foot (30.2 g/m²), 1.4 grams per square foot (15.1 g/m²) on each side, was prepared as described in Examples 9 and 10 of U.S. Patent 4,117,814. The tissue was removed from one side of a large sample of the film and placed on the surface of a laboratory table. Deionized water was sprayed on the surface of the film and a fibrous, non-woven fabric with a weight of 8.37 grams per square foot (90.2 g/m²) was placed on the tacky surface and pressed firmly with a hand rubber roll. After conditioning for one week at 72°F (22.2°C) and 50 percent relative humidity, a sample of the laminate was dried to 4 percent moisture content. The dry laminate was placed on a screen, 16 mesh, .023" diameter (0.58 mm) wire and run between a rubber covered steel roll (78 Durometer A hardness) and a steel roll at a pressure of approximately 50 psig (3.52 kg/cm²). The laminate had a softness rating of about 8—9.

Control 5 — Absence of drying

An absorbent film/tissue laminate was made by the technique described in Examples 9 and 10 of U.S. Patent 4,117,184. The weight of the laminate was approximately 6.5 grams per square foot (70.0 g/m²) with an absorbent film weight of 3.7 grams per square foot (39.9 g/m²) and a total tissue weight of 2.8 grams per square foot (30.2 g/m²), 1.4 grams per square foot (15.1 g/m²) on each side of the film. The laminate had about 14 percent moisture by weight.

The laminate was cut into 3-inch wide (7.62 cm) strips about 12 inches (30.5 cm) long. A strip of laminate was run between two matched, engraved steel rolls under 40 psig (2.82 kg/cm²) pressure at 20 feet (6.1 m) per minute. The pattern was perfectly reproduced in the laminate but the flexibility of the laminate was not increased as evidenced by visual observation. Two different, deeply engraved patterns were used with no evidence of increased flexibility in the laminate. This illustrates the fact that with a high moisture content laminate, there is no crushing of the film because the film remains flexible and does not break.

Control 6

The laminate described in Control 5 is run between a rubber covered steel roll (rubber hardness of about 50—60 Durometer A) and a 2-inch wide (5.1 cm) engraved pattern roll. Sufficient pressure was used to transfer the engraved roll pattern into the laminate. The flexibility of the laminate was not significantly increased by this action. Ten different engraved pattern rolls were tested with no significant improvement noted in flexibility.

### Example 15

The laminate described in Control 5 was placed on a fibrous belt that was heated from the underside by an infrared heating bar to a temperature of about 160°F—180°F (71.1°C—82.2°C). The laminate was exposed to the heated belt for about 20—30 seconds to reduce the moisture content and immediately run between the rolls described in Control 6. The engraved roll pattern was transferred to the laminate and the flexibility of the laminate was significantly increased. The laminate was soft and pliable with a hand similar to paper tissue.

### Example 16

Five engraved roll patterns were selected and tested according to Example 15. All of the patterns produced a significant increase in the flexibility of the laminate when the laminate was preheated and dried prior to running between the rolls.

### Example 17

An absorbent composition was made as follows:

|  | Parts by Wt. (dry) | Parts by Wt. (wet) |
|---|---|---|
| Acrylic Polymer Solution* | 94.4 | 374.6 |
| Polyoxyethylene Sorbitan monolaurate | 5.0 | 5.0 |
| Polyamidepolyamine/Epichlorohydrin resin | 0.6 | 14.4 |
| Deionized water to reach 20% solids | — | 106.0 |

\* Made according to Example 1 of U.S. Patent 4,076,673.

A glass plate 6 × 18 inches (15.2 cm × 45.7 cm) was precoated with a hard surface silicone release coating resin. The plate was preheated to 250°F (121°C) in a forced hot air oven and a 15 mil (0.38 mm) wet film of the absorbent composition was cast on the hot, release coated, glass plate and placed in the hot air oven for 1 minute. The plate was removed from the oven, the film lifted from the plate, and placed in a room at 50 percent relative humidity and 72°F (22.2°C) for 24 hours.

The film was placed on a large glass plate and sprayed with a light mist of deionized water to make the surface tacky. A piece of woven cotton textile (65 × 65 count) was immediately placed on the surface of the absorbent film and pressed firmly in position with a hard rubber roll. The one-sided laminate was placed with the film side up and a second piece of the cotton textile was adhered to the film surface as previously described.

The absorbent laminate was placed in a controlled environment at 75°F (23.9°C) and 50 percent relative humidity for 24 hours and then dried in a 200°F (93.3°C) oven to a moisture content of approximately 3 percent. The laminate was placed on a wire screen, 16 mesh, .023″ diameter (0.58 mm) wire and run between a rubber covered steel roll (Rubber hardness of 78 Durometer A) and a steel roll at a pressure of approximately 40 psig (2.82 kg/cm²) and roll speed of 20 ft/minute (6.10 m/min). The laminate flexibility was greatly increased. Originally it was stiff and boardy and after calendering, it is soft and pliable.

### Example 18

An absorbent film was obtained as described in Example 17. A piece of 1/8-inch thick (0.32 cm), flexible, open cell urethane foam of about 1.5 to 2.0 lb/ft³ (0.68—0.01 kg/0.028 m³) density was laminated to each surface of the film by the method described in Example 17. After 24 hours aging the moisture content of the laminate was reduced to about 2—3 percent by drying and the dry laminate was run between the calender rolls as described. The mechanical operation significantly increased the flexibility of the laminate from a rather stiff material to a soft flexible material.

### Claims

1. A flexible hydrophilic absorbent laminate comprising a central film consisting of a crosslinked water-swellable polyelectrolyte that is water-soluble in the salt form and a layer of wicking substrates bonded to both sides of said film, characterized in that
— said film is discontinuous, crushed and laminated to said wicking substrates, and
— said flexible absorbent laminate is flexible at low and high relative humidity and is obtainable by a process consisting in passing the laminate, if necessary dried to less than 8 wt.% residual moisture,

# 0 022 227

through a crushing zone wherein the said film breaks into a plurality of pieces which remain laminated to said substrates.

2. The absorbent laminate of claim 1 further characterized in that the polyelectrolyte is a lightly cross-linked carboxylated polyelectrolyte, obtainable by using an amount of 0.05—5% of cross-linking agents based on the weight of the polyelectrolyte.

3. The laminate of claim 1 wherein the film is constituted by an aerated film having a density ranging from 1.1 to 0.3 gram per cubic centimeter.

4. The laminate of claim 1 wherein the wicking substrates on the top and bottom each are selected from the group consisting of woven fabrics, non-woven fiber mats and polymeric foams.

5. An absorbent pad comprising an absorbent laminate, one or more layers of wicking substrates, a water impermeable bottom sheet and a water permeable face sheet characterized in that the absorbent laminate is as defined in any one of claims 1 to 4.

6. The absorbent pad of claim 5 wherein said water impermeable bottom sheet is polyethylene and said face sheet is a non-woven fiber mat.

7. A process for making the flexible laminate of claim 1 which comprises

(a) reducing the moisture content of a laminate of a film, prepared from a cross-linked, water-swellable polyelectrolyte that is water-soluble in the salt form, with wicking substrates to less than 8 percent by weight by passing said laminate through a drying zone; and

(b) passing said dried laminate through a crushing zone wherein said film breaks into a plurality of pieces which remain laminated to said substrates.

8. A process as in claim 7 characterized in that the film is prepared from a lightly cross-linked carboxylic polyelectrolyte, obtainable by using an amount of 0.05—5% of cross-linking agents based on the weight of the polyelectrolyte.

## Patentansprüche

1. Biegsames hydrophiles absorbierendes Laminat mit einer zentralen, aus einem vernetzten wasserquellbaren und in der Salzform wasserlöslichen Polyelektrolyten bestehenden Folie und einer Schicht dochtartiger Substrate, die beidseitig mit dieser Folie verklebt sind, dadurch gekennzeichnet, dass

— diese Folie diskontinuierlich, aufgebrochen und mit diesen dochtartigen Substraten laminiert ist und

— dieses flexible absorbierende Laminat bei niedriger und hoher relativer Feuchte biegsam und nach einem Verfahren erhältlich ist, bei dem das gegebenenfalls auf weniger als 8 Gew.-% Restfeuchte getrocknete Laminat durch eine Brechzone geführt wird, wo diese Folie in zahlreiche Stücke aufbricht, die mit jenen Substraten laminiert bleiben.

2. Absorbierendes Laminat nach Anspruch 1, ferner dadurch gekennzeichnet, dass der Polyelektrolyt ein leicht vernetzter carboxylierter Polyelektrolyt ist, der unter Einsatz einer Menge von 0,05—5% Vernetzungsmittel, bezogen auf das Gewicht des Polyelektrolyten, erhältlich ist.

3. Laminat nach Anspruch 1, worin die Folie aus einer Schaumstoffolie mit einer Dichte im Bereich von 1,1 bis 0,3 g/cm³ besteht.

4. Laminat nach Anspruch 1, worin die dochtartigen Substrate auf der Ober- und Unterseite unter Geweben, Faservliesmatten und polymeren Schäumen ausgewählt sind.

5. Saugkissen, bestehend aus einem absorbierenden Laminat, einer oder mehreren Schichten dochtartiger Substrate, einem wasserundurchlässigen Unterlageblatt und einem wasserdurchlässigen Deckblatt, dadurch gekennzeichnet, dass das absorbierende Laminat wie in einem der Ansprüche 1 bis 4 definiert ausgebildet ist.

6. Saukgissen nach Anspruch 5, worin dieses wasserundurchlässige Unterlageblatt aus Polyäthylen und dieses Deckblatt aus einer Faservliesmatte besteht.

7. Verfahren zur Herstellung des biegsamen Laminats nach Anspruch 1, dadurch gekennzeichnet, dass man

(a) den Feuchtegehalt eines Laminats aus einer aus einem vernetzten, wasserquellbaren und in der Salzform wasserlöslichen Polyelektrolyten hergestellten Folie und dochtartigen Substraten auf weniger als 8 Gewichtsprozent verringert, indem man dieses Laminat durch eine Trockenzone führt, und

(b) dieses getrocknete Laminat durch eine Brechzone leitet, wo diese Folie in zahlreiche Stücke aufbricht, die mit jenen Substraten laminiert bleiben.

8. Verfahren nach Anspruch 7, dadurch gekennzeichnet, dass die Folie aus einem leicht vernetzten Carboxylpolyelektrolyten hergestellt wird, der unter Einsatz einer Menge von 0,05—5% Vernetzungsmittel, bezogen auf das Gewicht des Polyelektrolyten, erhältlich ist.

## Revendications

1. Stratifié absorbant hydrophile flexible comprenant un film central constitué par un polyélectrolyte réticulé gonflable à l'eau qui est hydrosoluble sous la forme de sel et une couche de substrats capables de s'imbiber liés aux deux côtés dudit film, caractérisé en ce que

8

— ledit film est discontinu, broyé et stratifié auxdits substrats capables de s'imbiber, et

— ledit stratifié absorbant flexible est flexible à des taux d'humidité relative faibles et élevés et peut être obtenu par un procédé consistant en un passage du stratifié, éventuellement séché jusqu'à une teneur en eau résiduelle inférieure à 8% en poids, à travers une zone de broyage dans laquelle ledit film se casse en un grand nombre de morceaux qui demeurent stratifiés auxdits substrats.

2. Stratifié absorbant selon la revendication 1, caractérisé en outre en ce que le polyélectrolyte est un polyélectrolyte carboxylé légèrement réticulé que l'on peut obtenir en utilisant une quantité d'agents réticulants de 0,05 à 5% par rapport au poids du polyélectrolyte.

3. Stratifié selon la revendication 1, caractérisé en ce que le film est constitué par un film aéré de masse volumique comprise entre 1,1 et 0,3 grammes par centimètre cube.

4. Stratifié selon la revendication 1, caractérisé en ce que les substrats capables de s'imbiber du dessus et du fond sont choisis chacun dans le groupe constitué par les tissus tissés, les matelas de fibres non tissées et les mousses polymères.

5. Matelas absorbant comprenant un stratifié absorbant, une ou plusieurs couches de substrats capables de s'imbiber, une feuille de fond imperméable à l'eau et une feuille de dessus perméable à l'eau, caractérisé en ce que le stratifié absorbant est tel que défini dans l'une quelconque des revendications 1 à 4.

6. Matelas absorbant selon la revendication 5, caractérisé en ce que ladite feuille de fond imperméable à l'eau est du polyéthylène et ladite feuille de dessus est un matelas de fibres non tissées.

7. Procédé de fabrication du stratifié flexible selon la revendication 1 qui comprend

(a) une réduction de la teneur en eau d'un stratifié d'un film préparé à partir d'un polyélectrolyte réticulé gonflable à l'eau qui est hydrosoluble sous forme de sel et de substrats capables de s'imbiber, jusqu'à moins de 8 pour cent en poids, en faisant passer ledit stratifié à travers une zone de séchage; et

(b) un passage dudit stratifié séché à travers une zone de broyage dans laquelle ledit film se casse en un grand nombre de morceaux qui demeurent stratifiés auxdits substrats.

8. Procédé selon la revendication 7, caractérisé en ce que le film est préparé à partir d'un polyélectrolyte carboxylé légèrement réticulé que l'on peur obtenir en utilisant une quantité d'agent réticulants de 0,05 à 5% par rapport au poids du polyélectrolyte.

FIG. 1

FIG. 2